# EUROPEAN PATENT SPECIFICATION

(11) **EP 4 267 236 B1**
(45) Date of publication and mention of the grant of the patent: **24.06.2026**
(21) Application number: 21851628.4
(22) Date of filing: 22.12.2021
(51) Int. Cl.: A61N 1/32, A61N 1/36, A61N 1/40, C12N 15/87, A61P 35/00, C12N 13/00, C12M 1/42

(54) **USING ALTERNATING ELECTRIC FIELDS IN GENE THERAPY**
VERWENDUNG VON ELEKTRISCHEN WECHSELFELDERN IN DER GENTHERAPIE
UTILISISATION DE CHAMPS ÉLECTRIQUES ALTERNATIFS EN THÉRAPIE GÉNIQUE

(30) Priority: 24.12.2020 US 202063130449 P
(43) Date of publication of application: 01.11.2023
(73) Proprietor: Novocure GmbH, 6340 Baar (CH)
(72) Inventor: VOLOSHIN-SELA, Tali, 31905 Sha'ar HaCarmel, Haifa (IL); AVIGDOR, Lilach, 31905 Sha'ar HaCarmel (IL); PORAT, Yaara, 31905 Haifa (IL); MUMBLAT, Helena, 31905 Haifa (IL)
(74) Representative: Carpmaels & Ransford LLP
(86) International application number: PCT/IB2021/062198
(87) International publication number: WO 2022/137171

(56) References cited:
- WO-A1-2020/047387
- US-A1- 2019 336 757
- US-A1- 2020 009 376
- US-A1- 2020 121 728
- US-A1- 2020 308 560
- No Search

## Description

### BACKGROUND

It has previously been shown that when cells are exposed to an alternating electric field (AEF) in specific frequency ranges while the cell is undergoing mitosis, the AEF can disrupt the mitosis process and cause apoptosis. This phenomenon has been successfully used to treat tumors (e.g. glioblastoma, mesothelioma, etc.) as described in U.S. Pat. Nos. 7,016,725 and 7,565,205. And in the context of treating tumors, these alternating electric fields are referred to as "TTFields" (or "Tumor Treating Fields"). One of the reasons why TTFields therapy is well-suited for treating tumors is that TTFields selectively disrupt dividing cells during mitosis, and apparently have no effect on cells that are not dividing. And because tumor cells divide much more often than other cells in a person's body, applying TTFields to a subject will selectively attack the tumor cells while leaving the other cells unharmed. The same phenomenon has also been successfully shown to be useful for destroying bacteria, as described in U.S. Pat. No. 9,750,934. And here again, one of the reasons why this approach is well-suited for destroying bacteria is that bacteria cells divide much more rapidly than other cells in a person's body.

Alternating electric fields have also been show to increase permeability of cell membranes. Much work has been done on viral gene therapy and many ongoing clinical trials have been conducted with virus-based therapies for cancer. No efficient and specific viral therapy is currently in use in cancer patients. The combination of using alternating electric fields to help deliver a therapeutic into a cell for treating cancer in a subject is needed.

US 2020/0009376 Al describes certain substances (e.g., large molecules) that ordinarily cannot traverse the cell membrane of cells. These substances can be introduced into cells by applying an alternating electric field to the cell for a period of time, wherein the frequency of the alternating electric field is selected so that application of the alternating electric field increases permeability of the cell membrane. Once the permeability of the cell membrane has been increased, the substance is able to cross the cell membrane. This approach is particularly useful in the context of cancer
cells ( e.g., glioblastoma ).

US 2020/0121728 Al provides approaches for eliminating residual pluripotent stem cells that may remain in a batch of differentiated progeny cells in the context of a stem cell-based therapy in which differentiated cells are derived from pluripotent stem cells. This advantageously prevents the formation of teratoma tumors when the differentiated cells are eventually used for the therapy. This can be accomplished by exposing the batch of differentiated progeny cells and the residual pluripotent stem cells to an alternating electric field for a period of time. The frequency and field strength of the alternating electric field are such that the pluripotent stem cells die off as a result of exposure to the alternating electric fields, while the differentiated cells remain substantially unharmed. This results in a purified batch of differentiated progeny cells that is rendered safe for use in the stem cell-based therapy

US 2019/0336757 A1 provides systems, methods, and apparatus for electroporation, which may include an applicator; an endoscope, trocar or the like; a generator; and a drug delivery device. The applicator may include a control portion, an insertion tube connected to the control portion, an actuator engaged with the control portion, and a plurality of electrodes comprising a first electrode having a first tip and a second electrode having a second tip. The plurality electrodes may be configured to move between a retracted position and a deployed position in response to actuation by the actuator. A distance between the first tip of the first electrode and the second tip of the second electrode may be greater in the deployed position than in the retracted position. Various treatment methods are also provided.

US 2020/0308560 A1 provides systems, methods, and compositions for targeting nucleic acids. In particular, the invention provides non-naturally occurring or engineered RNA-targeting systems comprising a novel RNA-targeting CRISPR effector protein and at least one

### SUMMARY OF THE PRESENT INVENTION

The present invention is defined in the appended claims. Aspects, embodiments, examples and methods described hereinafter are only of exemplary nature and presented for better understanding the present invention, which scope is defined by the appended claims. Certain methods of treating, killing cells, and reducing the viability of cells are described with reference to the vector of the present invention. Whilst no claim is directed to these methods *per se,* the vector is capable of being used and is intended to be used in such methods.

Combining virus-based therapy or virus over-expressing proteins for theragnostic delineation of cancer cells from normal tissue with TTFields can be both specific and enhanced in cancer cells compared to healthy tissue or circulating cells.

The difference in effect of TTFields application in viral load in cancer cells compared to healthy cells indicates that viral gene therapy and/or viruses carrying a detectable maker to delineate cancer cells from normal tissue can be used in combination with TTFields to allow a specific and enhanced effect in cancer cells and not in healthy cells.

Enhancing viral load in cancer cells while decreasing viral load in healthy cells can impact treatment with viral gene therapy and also provide a better tool to visualize cancer tissue or metastatic cells that have been infected with a vector expressing a detectable marker.

Disclosed are methods for transporting a composition across a cell membrane of a cell, the method comprising applying an alternating electric field to the cell for a period of time, wherein application of the alternating electric field increases permeability of the cell membrane; and introducing the composition to the cell, wherein the increased permeability of the cell membrane enables the composition to cross the cell membrane. In some aspects, the composition can be a vector, therapeutic, detectable label, or combination thereof.

Disclosed are methods for transporting a vector across a cell membrane of a cell, the method comprising applying an alternating electric field to the cell for a period of time, wherein application of the alternating electric field increases permeability of the cell membrane; and introducing the vector to the cell, wherein the increased permeability of the cell membrane enables the vector to cross the cell membrane.

Disclosed are methods for reducing the viability of a cell, the method comprising: applying a first alternating electric field at a first frequency to the cell for a first period of time, wherein application of the first alternating electric field at the first frequency to the cell for the first period of time increases permeability of the cell membrane of the cell; introducing a composition to the cell, wherein the increased permeability of the cell membrane enables the composition to cross the cell membrane; and applying a second alternating electric field at a second frequency to the cell for a second period of time, wherein the second frequency is different from the first frequency, and wherein the second alternating electric field at the second frequency reduces viability of the cell. In some aspects, the composition can be a vector, therapeutic, detectable label, or combination thereof. In some aspects, the cell is a tumor or cancer cell.

Disclosed are methods of reducing the viability of a cell comprising applying a first alternating electric field at a first frequency to the cell for a period of time, wherein application of the first alternating electric field increases permeability of the cell membrane; introducing a vector to the cell, wherein the increased permeability of the cell membrane enables the vector to cross the cell membrane, wherein the vector comprises a nucleic acid sequence capable of encoding a therapeutic agent and wherein, once the vector crosses the cell membrane, the vector encodes the therapeutic agent, wherein the presence of the therapeutic agent reduces the viability of the cell. In some aspects, the cell is a tumor or cancer cell.

Disclosed are methods of detecting a tumor or cancer cell comprising applying a first alternating electric field at a first frequency to the tumor or cancer cell for a period of time, wherein application of the first alternating electric field increases permeability of the tumor or cancer cell membrane; introducing a vector to the tumor or cancer cell, wherein the increased permeability of the tumor or cancer cell membrane enables the vector to cross the tumor or cancer cell membrane, wherein the vector comprises a nucleic acid sequence capable of encoding a selectable marker and wherein, once the vector crosses the tumor or cancer cell membrane, the vector encodes the selectable marker; and detecting the selectable marker, wherein the presence of the selectable marker indicates the presence of a tumor or cancer cell.

Disclosed are methods of detecting and treating a tumor or cancer cell comprising: applying a first alternating electric field at a first frequency to the tumor or cancer cell for a period of time, wherein application of the alternating electric field increases permeability of the tumor or cancer cell membrane; introducing a vector to the tumor or cancer cell, wherein the increased permeability of the tumor or cancer cell membrane enables the vector to cross the tumor or cancer cell membrane, wherein the vector comprises nucleic acid sequences capable of encoding a therapeutic agent and a selectable marker and wherein, once the vector enters the cell the vector encodes the therapeutic agent and the selectable marker; and detecting the selectable marker, wherein the presence of the selectable marker indicates the presence of a tumor or cancer cell and wherein the therapeutic agent encoded by the vector induces apoptosis of the tumor or cancer cell.

Additional advantages of the disclosed method and compositions will be set forth in part in the description which follows, and in part will be understood from the description, or may be learned by practice of the disclosed method and compositions. It is to be understood that both the foregoing general description and the following detailed description are exemplary and explanatory only and are not restrictive of the invention which is defined by the appended claims.

### BRIEF DESCRIPTION OF THE DRAWINGS

The accompanying drawings, which are incorporated in and constitute a part of this specification, illustrate several embodiments of the disclosed method and compositions and together with the description, serve to explain the principles of the disclosed method and compositions.
Figure 1 shows the effects of 24 hours TTFields application to A549 cells infected with the PR8 virus during the viral infection and proliferation.
Figure 2 shows the effects of 48 hours TTFields application to A549 cells infected with the PR8 virus during the viral infection and proliferation.

### DETAILED DESCRIPTION

The disclosed method and compositions may be understood more readily by reference to the following detailed description of particular embodiments and the Examples included therein and to the Figures and their previous and following description.

It is to be understood that the disclosed method and compositions are not limited to specific synthetic methods, specific analytical techniques, or to particular reagents unless otherwise specified, and, as such, may vary. It is also to be understood that the terminology used herein is for the purpose of describing particular embodiments only and is not intended to be limiting.

Disclosed are materials, compositions, and components that can be used for, can be used in conjunction with, can be used in preparation for, or are products of the disclosed method and compositions. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. Thus, if a class of molecules A, B, and C are disclosed as well as a class of molecules D, E, and F and an example of a combination molecule, A-D is disclosed, then even if each is not individually recited, each is individually and collectively contemplated. Thus, is this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-E are specifically contemplated and should be considered disclosed from disclosure of A, B, and C; D, E, and F; and the example combination A-D. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods, and that each such combination is specifically contemplated and should be considered disclosed.

### A. Definitions

It is understood that the disclosed method and compositions are not limited to the particular methodology, protocols, and reagents described as these may vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a", "an", and "the" include plural reference unless the context clearly dictates otherwise. Thus, for example, reference to "a vector" includes a single or a plurality of such vectors, reference to "the vector" is a reference to one or more vectors and equivalents thereof known to those skilled in the art, and so forth.

As used herein, a "target site" is a specific site or location within or present on a subject or patient. For example, a "target site" can refer to, but is not limited to a cell (e.g. a cancer cell), population of cells, organ, tissue, or a tumor. Thus, the phrase "target cell" can be used to refer to target site, wherein the target site is a cell. In some aspects, organs that can be target sites include, but are not limited to, lung, brain, pancreas, abdominal organs (e.g. stomach, intestine), ovary, breast, uterus, prostate, bladder, liver, colon, or kidney. In some aspects, a cell or population of cells that can be target site or target cell include, but are not limited to, lung cells, brain cells, pancreatic cells, abdominal cells, ovarian cells, liver cells, colon cells, or kidney cells. In some aspects, a "target site" can be a tumor target site. In some aspects, a "target site" can be a virus-infected cell. In some aspects, a "target cell" can be a cancer. In some aspects, a "target cell" can be a virus-infected cell.

A "tumor target site" is a site or location within or present on a subject or patient that comprises or is adjacent to one or more cancer cells, previously comprised one or more tumor cells, or is suspected of comprising one or more tumor cells. For example, a tumor target site can refer to a site or location within or present on a subject or patient that is prone to metastases. Additionally, a target site or tumor target site can refer to a site or location of a resection of a primary tumor within or present on a subject or patient. Additionally, a target site or tumor target site can refer to a site or location adjacent to a resection of a primary tumor within or present on a subject or patient.

As used herein, an "alternating electric field" or "alternating electric fields" refers to a very-low-intensity, directional, intermediate-frequency alternating electrical fields delivered to a subject, a sample obtained from a subject or to a specific location within a subject or patient (e.g. a target site such as a cell). In some aspects, the alternating electrical field can be in a single direction or multiple directional. In some aspects, alternating electric fields can be delivered through two pairs of transducer arrays that generate perpendicular fields within the target site. For example, for the Optune^{™} system (an alternating electric fields delivery system) one pair of electrodes is located to the left and right (LR) of the target site, and the other pair of electrodes is located anterior and posterior (AP) to the target site. Cycling the field between these two directions (i.e., LR and AP) ensures that a maximal range of cell orientations is targeted.

As used herein, an "alternating electric field" applied to a tumor target site can be referred to as a "tumor treating field" or "TTField." TTFields have been established as an anti-mitotic cancer treatment modality because they interfere with proper micro-tubule assembly during metaphase and eventually destroy the cells during telophase, cytokinesis, or subsequent interphase. TTFields target solid tumors and is described in U.S. Pat. No. 7,565,205 for its teaching of TTFields

In-vivo and in-vitro studies show that the efficacy of TTFields therapy increases as the intensity of the electrical field increases. Therefore, optimizing array placement on a subject to increase the intensity in the target site or target cell is standard practice for the Optune system. Array placement optimization may be performed by "rule of thumb" (e.g., placing the arrays on the subject as close to the target site or target cell as possible), measurements describing the geometry of the patient's body, target site dimensions, and/or target site or cell location. Measurements used as input may be derived from imaging data. Imaging data is intended to include any type of visual data, such as for example, single-photon emission computed tomography (SPECT) image data, x-ray computed tomography (x-ray CT) data, magnetic resonance imaging (MRI) data, positron emission tomography (PET) data, data that can be captured by an optical instrument (e.g., a photographic camera, a charge-coupled device (CCD) camera, an infrared camera, etc.), and the like. In certain implementations, image data may include 3D data obtained from or generated by a 3D scanner (e.g., point cloud data). Optimization can rely on an understanding of how the electrical field distributes within the target site or target cell as a function of the positions of the array and, in some aspects, take account for variations in the electrical property distributions within the heads of different patients.

The term "subject" refers to the target of administration, e.g. an animal. Thus, the subject of the disclosed methods can be a vertebrate, such as a mammal. For example, the subject can be a human. The term does not denote a particular age or sex. Subject can be used interchangeably with "individual" or "patient." For example, the subject of administration can mean the recipient of the alternating electrical field.

By "treat" is meant to administer or apply a therapeutic, such as alternating electric fields and a vector, to a subject, such as a human or other mammal (for example, an animal model), that has an infection (e.g. viral) or disease (e.g. cancer) or has an increased susceptibility for developing an infection or disease, in order to prevent or delay a worsening of the effects of the disease or infection, or to partially or fully reverse the effects of the infection or disease. For example, treating a subject having cancer can comprise delivering a therapeutic to a cell in the subject.

By "prevent" is meant to minimize or decrease the chance that a subject develops an infection or disease.

As used herein, the terms "administering" and "administration" refer to any method of providing a therapeutic, such as an antiviral agent or anti-cancer therapeutic, to a target site or subject. Such methods are well known to those skilled in the art and include, but are not limited to: oral administration, transdermal administration, administration by inhalation, nasal administration, topical administration, intravaginal administration, ophthalmic administration, intraaural administration, intracerebral administration, rectal administration, sublingual administration, buccal administration, and parenteral administration, including injectable such as intravenous administration, intra-arterial administration, intramuscular administration, and subcutaneous administration. Administration can be continuous or intermittent. In various aspects, a preparation can be administered therapeutically; that is, administered to treat an existing disease or condition. In further various aspects, a preparation can be administered prophylactically; that is, administered for prevention of a disease or condition. In an aspect, the skilled person can determine an efficacious dose, an efficacious schedule, or an efficacious route of administration so as to treat a subject. In some aspects, administering comprises exposing or applying. Thus, in some aspects, exposing a target site or subject to alternating electrical fields or applying alternating electrical fields to a target site or subject means administering alternating electrical fields to the target site or subject.

"Optional" or "optionally" means that the subsequently described event, circumstance, or material may or may not occur or be present, and that the description includes instances where the event, circumstance, or material occurs or is present and instances where it does not occur or is not present.

Ranges may be expressed herein as from "about" one particular value, and/or to "about" another particular value. When such a range is expressed, also specifically contemplated and considered disclosed is the range from the one particular value and/or to the other particular value unless the context specifically indicates otherwise. Similarly, when values are expressed as approximations, by use of the antecedent "about," it will be understood that the particular value forms another, specifically contemplated embodiment that should be considered disclosed unless the context specifically indicates otherwise. It will be further understood that the endpoints of each of the ranges are significant both in relation to the other endpoint, and independently of the other endpoint unless the context specifically indicates otherwise. Finally, it should be understood that all of the individual values and sub-ranges of values contained within an explicitly disclosed range are also specifically contemplated and should be considered disclosed unless the context specifically indicates otherwise. The foregoing applies regardless of whether in particular cases some or all of these embodiments are explicitly disclosed.

Unless defined otherwise, all technical and scientific terms used herein have the same meanings as commonly understood by one of skill in the art to which the disclosed method and compositions belong. Although any methods and materials similar or equivalent to those described herein can be used in the practice or testing of the present method and compositions, the particularly useful methods, devices, and materials are as described.

Throughout the description and claims of this specification, the word "comprise" and variations of the word, such as "comprising" and "comprises," means "including but not limited to," and is not intended to exclude, for example, other additives, components, integers or steps. In particular, in methods stated as comprising one or more steps or operations it is specifically contemplated that each step comprises what is listed (unless that step includes a limiting term such as "consisting of"), meaning that each step is not intended to exclude, for example, other additives, components, integers or steps that are not listed in the step.

### B. Vectors

Disclosed are vectors comprising a nucleic acid sequence capable of encoding a sequence of interest. The disclosed methods can comprise the administration of one or more of the vectors described herein.

In some aspects, the vectors for use in the methods disclosed herein can be expression vectors. The term "expression vector" includes any vector, (e.g., a plasmid, cosmid, phagemid, phage, virus) containing a gene construct in a form suitable for expression by a cell (e.g., linked to a transcriptional control element).

The vectors for use in the methods disclosed herein can be non-viral vectors or viral vectors. In some aspects, the vector can be a viral vector. For example, the viral vector can be an adeno-associated viral vector, an adeno-associated virus, a lentivirus or a herpes virus. In some aspects, the vector can be a non-viral vector, such as a DNA based vector (e.g. plasmid, cosmid, phagemid).

### 1. Viral and Non-Viral Vectors

There are a number of vectors and methods which can be used to deliver the disclosed vectors, either *in vitro* or *in vivo.* These methods and compositions can largely be broken down into two classes: viral based delivery systems and non-viral based delivery systems. For example, a nucleic acid capable of encoding a sequence of interest can be delivered across a cell membrane of a cell, the method comprising: applying an alternating electric field to the cell for a period of time, wherein application of the alternating electric field increases permeability of the cell membrane; and introducing the vector to the cell, wherein the increased permeability of the cell membrane enables the vector to cross the cell membrane. Such vectors can be in the form of plasmids, viral vectors, viral nucleic acids, phage nucleic acids, phages, cosmids, or cationic liposomes. In certain cases, the methods can be modified to specifically function with large DNA molecules. Further, these methods can be used to target certain diseases and cell populations by using the targeting characteristics of a carrier.

Expression vectors can be any nucleotide construct used to deliver genes or gene fragments into cells (e.g., a plasmid), or as part of a general strategy to deliver genes or gene fragments, e.g., as part of recombinant retrovirus or adenovirus (Ram et al. Cancer Res. 53:83-88, (1993)). For example, disclosed herein are expression vectors comprising a nucleic acid sequence capable of encoding a sequence of interest, such as a cancer therapeutic.

In some aspects, the disclosed vectors comprise control elements. The "control elements" present in an expression vector are those non-translated regions of the vectorenhancers, promoters, 5' and 3' untranslated regions--which interact with host cellular proteins to carry out transcription and translation. Such elements may vary in their strength and specificity. Depending on the vector system and host utilized, any number of suitable transcription and translation elements, including constitutive and inducible promoters, may be used. For example, when cloning in bacterial systems, inducible promoters such as the hybrid lacZ promoter of the pBLUESCRIPT phagemid (Stratagene, La Jolla, Calif.) or pSPORT1 plasmid (Gibco BRL, Gaithersburg, Md.) and the like may be used. If it is necessary to generate a cell line that contains multiple copies of the sequence encoding a sequence of interest, vectors based on SV40 or EBV may be advantageously used with an appropriate selectable marker.

"Enhancer" generally refers to a sequence of DNA that functions at no fixed distance from the transcription start site and can be either 5' (Laimins, L. et al., Proc. Natl. Acad. Sci. 78: 993 (1981)) or 3' (Lusky, M.L., et al., Mol. Cell Bio. 3: 1108 (1983)) to the transcription unit. Furthermore, enhancers can be within an intron (Banerji, J.L. et al., Cell 33: 729 (1983)) as well as within the coding sequence itself (Osborne, T.F., et al., Mol. Cell Bio. 4: 1293 (1984)). They are usually between 10 and 300 bp in length, and they function in cis. Enhancers function to increase transcription from nearby promoters. Enhancers also often contain response elements that mediate the regulation of transcription. Promoters can also contain response elements that mediate the regulation of transcription. Enhancers often determine the regulation of expression of a gene. While many enhancer sequences are now known from mammalian genes (globin, elastase, albumin, α-fetoprotein and insulin), typically one will use an enhancer from a eukaryotic cell virus for general expression. Preferred examples are the SV40 enhancer on the late side of the replication origin (bp 100-270), the cytomegalovirus early promoter enhancer, the polyoma enhancer on the late side of the replication origin, and adenovirus enhancers.

The promoter or enhancer may be specifically activated either by light or specific chemical events which trigger their function. Systems can be regulated by reagents such as tetracycline and dexamethasone. There are also ways to enhance viral vector gene expression by exposure to irradiation, such as gamma irradiation, or alkylating chemotherapy drugs.

In some aspects, the promoter or enhancer region can act as a constitutive promoter or enhancer to maximize expression of the polynucleotides. In certain constructs the promoter or enhancer region be active in all eukaryotic cell types, even if it is only expressed in a particular type of cell at a particular time.

Expression vectors for use in the methods disclosed in eukaryotic host cells (yeast, fungi, insect, plant, animal, human or nucleated cells) may also contain sequences necessary for the termination of transcription which may affect mRNA expression. These regions are transcribed as polyadenylated segments in the untranslated portion of the mRNA encoding tissue factor protein. The 3' untranslated regions also include transcription termination sites. It is preferred that the transcription unit also contains a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs. In certain transcription units, the polyadenylation region is derived from the SV40 early polyadenylation signal and consists of about 400 bases.

The expression vectors for use in the methods disclosed can include a nucleic acid sequence encoding a marker product. This marker product can be used to determine if the gene has been delivered to the cell and once delivered is being expressed. Marker genes can include, but are not limited to the E. coli lacZ gene, which encodes β-galactosidase, and the gene encoding the green fluorescent protein.

In some embodiments the marker may be a selectable marker. Examples of suitable selectable markers for mammalian cells are dihydrofolate reductase (DHFR), thymidine kinase, neomycin, neomycin analog G418, hydromycin, and puromycin. For in vitro uses, cell comprising the vectors after using the methods described herein can be isolated. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are CHO DHFR-cells and mouse LTK-cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media.

Another type of selection that can be used with the composition and methods disclosed herein is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, (Southern P. and Berg, P., J. Molec. Appl. Genet. 1: 327

(1982)), mycophenolic acid, (Mulligan, R.C. and Berg, P. Science 209: 1422 (1980)) or hygromycin, (Sugden, B. et al., Mol. Cell. Biol. 5: 410-413 (1985)). The three examples employ bacterial genes under eukaryotic control to convey resistance to the appropriate drug G418 or neomycin (geneticin), xgpt (mycophenolic acid) or hygromycin, respectively. Others include the neomycin analog G418 and puramycin.

As used herein, plasmid or viral vectors are agents that transport a nucleic acid sequence, such as a nucleic acid sequence capable of encoding a sequence of interest (e.g. one or more proteins or peptides of interest) into the cell without degradation and include a promoter yielding expression of the gene in the cells into which it is delivered. In some embodiments the vectors are viral vectors. Viral vectors are, for example, Adenovirus, Adeno-associated virus, Herpes virus, Vaccinia virus, Polio virus, lentivirus (AIDS virus), neuronal trophic virus, Sindbis and other RNA viruses, including these viruses with the HIV backbone. Also preferred are any viral families which share the properties of these viruses which make them suitable for use as vectors. Retroviruses include Murine Maloney Leukemia virus, MMLV, and retroviruses that express the desirable properties of MMLV as a vector. Retroviral vectors are able to carry a larger genetic payload, i.e., a transgene or marker gene, than other viral vectors, and for this reason are a commonly used vector. However, they are not as useful in non-proliferating cells. Adenovirus vectors are relatively stable and easy to work with, have high titers, and can be delivered in aerosol formulation, and can transfect non-dividing cells. Pox viral vectors are large and have several sites for inserting genes, they are thermostable and can be stored at room temperature. A preferred embodiment is a viral vector which has been engineered so as to suppress the immune response of the host organism, elicited by the viral antigens. Preferred vectors of this type will carry coding regions for Interleukin 8 or 10.

Viral vectors can have higher transaction abilities (i.e., ability to introduce genes) than chemical or physical methods of introducing genes into cells. Typically, viral vectors contain, nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promoter cassette is inserted into the viral genome in place of the removed viral DNA. Constructs of this type can carry up to about 8 kb of foreign genetic material. The necessary functions of the removed early genes are typically supplied by cell lines which have been engineered to express the gene products of the early genes in trans.

Retroviral vectors, in general, are described by Verma, I.M., Retroviral vectors for gene transfer. In Microbiology, Amer. Soc. for Microbiology, pp. 229-232, Washington, (1985). Examples of methods for using retroviral vectors for gene therapy are described in U.S. Patent Nos. 4,868,116 and 4,980,286; PCT applications WO 90/02806 and WO 89/07136; and Mulligan, (Science 260:926-932 (1993)).

A retrovirus is essentially a package which has packed into it nucleic acid cargo. The nucleic acid cargo carries with it a packaging signal, which ensures that the replicated daughter molecules will be efficiently packaged within the package coat. In addition to the package signal, there are a number of molecules which are needed in cis, for the replication, and packaging of the replicated virus. Typically a retroviral genome contains the gag, pol, and env genes which are involved in the making of the protein coat. It is the gag, pol, and env genes which are typically replaced by the foreign DNA that it is to be transferred to the target cell. Retrovirus vectors typically contain a packaging signal for incorporation into the package coat, a sequence which signals the start of the gag transcription unit, elements necessary for reverse transcription, including a primer binding site to bind the tRNA primer of reverse transcription, terminal repeat sequences that guide the switch of RNA strands during DNA synthesis, a purine rich sequence 5' to the 3' LTR that serves as the priming site for the synthesis of the second strand of DNA synthesis, and specific sequences near the ends of the LTRs that enable the insertion of the DNA state of the retrovirus to insert into the host genome. This amount of nucleic acid is sufficient for the delivery of a one to many genes depending on the size of each transcript. It is preferable to include either positive or negative selectable markers along with other genes in the insert.

Since the replication machinery and packaging proteins in most retroviral vectors have been removed (gag, pol, and env), the vectors are typically generated by placing them into a packaging cell line. A packaging cell line is a cell line which has been transfected or transformed with a retrovirus that contains the replication and packaging machinery but lacks any packaging signal. When the vector carrying the sequence of interest is transfected into these cell lines, the vector containing the sequence of interest is replicated and packaged into new retroviral particles, by the machinery provided in cis by the helper cell. The genomes for the machinery are not packaged because they lack the necessary signals.

The construction of replication-defective adenoviruses has been described (Berkner et al., J. Virology 61:1213-1220 (1987); Massie et al., Mol. Cell. Biol. 6:2872-2883 (1986); Haj-Ahmad et al., J. Virology 57:267-274 (1986); Davidson et al., J. Virology 61:1226-1239 (1987); Zhang "Generation and identification of recombinant adenovirus by liposome-mediated transfection and PCR analysis" BioTechniques 15:868-872 (1993)). The benefit of the use of these viruses as vectors is that they are limited in the extent to which they can spread to other cell types, since they can replicate within an initial infected cell but are unable to form new infectious viral particles. Recombinant adenoviruses have been shown to achieve high efficiency gene transfer after direct, in vivo delivery to airway epithelium, hepatocytes, vascular endothelium, CNS parenchyma and a number of other tissue sites (Morsy, J. Clin. Invest. 92:1580-1586 (1993); Kirshenbaum, J. Clin. Invest. 92:381-387 (1993); Roessler, J. Clin. Invest. 92:1085-1092 (1993); Moullier, Nature Genetics 4:154-159 (1993); La Salle, Science 259:988-990 (1993); Gomez-Foix, J. Biol. Chem. 267:25129-25134 (1992); Rich, Human Gene Therapy 4:461-476 (1993); Zabner, Nature Genetics 6:75-83 (1994); Guzman, Circulation Research 73:1201-1207 (1993); Bout, Human Gene Therapy 5:3-10 (1994); Zabner, Cell 75:207-216 (1993); Caillaud, Eur. J. Neuroscience 5:1287-1291 (1993); and Ragot, J. Gen. Virology 74:501-507 (1993)). Recombinant adenoviruses achieve gene transduction by binding to specific cell surface receptors, after which the virus is internalized by receptor-mediated endocytosis, in the same manner as wild type or replication-defective adenovirus (Chardonnet and Dales, Virology 40:462-477 (1970); Brown and Burlingham, J. Virology 12:386-396 (1973); Svensson and Persson, J. Virology 55:442-449 (1985); Seth, et al., J. Virol. 51:650-655 (1984); Seth, et al., Mol. Cell. Biol., 4:1528-1533 (1984); Varga et al., J. Virology 65:6061-6070 (1991); Wickham et al., Cell 73:309-319 (1993)).

A viral vector for use in the methods disclosed can be one based on an adenovirus which has had the E1 gene removed and these virons are generated in a cell line such as the human 293 cell line. Optionally, both the E1 and E3 genes are removed from the adenovirus genome.

Another type of viral vector for use in the methods disclosed that can be used to introduce the polynucleotides of the invention into a cell is based on an adeno-associated virus (AAV). This defective parvovirus is a preferred vector because it can infect many cell types and is nonpathogenic to humans. AAV type vectors can transport about 4 to 5 kb and wild type AAV is known to stably insert into chromosome 19. Vectors which contain this site specific integration property are preferred. An especially preferred embodiment of this type of vector is the P4.1 C vector produced by Avigen, San Francisco, CA, which can contain the herpes simplex virus thymidine kinase gene, HSV-tk, or a marker gene, such as the gene encoding the green fluorescent protein, GFP.

In another type of AAV virus for use in the methods disclosed, the AAV contains a pair of inverted terminal repeats (ITRs) which flank at least one cassette containing a promoter which directs cell-specific expression operably linked to a heterologous gene. Heterologous in this context refers to any nucleotide sequence or gene which is not native to the AAV or B19 parvovirus. Typically the AAV and B19 coding regions have been deleted, resulting in a safe, noncytotoxic vector. The AAV ITRs, or modifications thereof, confer infectivity and site-specific integration, but not cytotoxicity, and the promoter directs cell-specific expression. United States Patent No. 6,261,834 shows material related to the AAV vector.

Other useful systems include, for example, replicating and host-restricted non-replicating vaccinia virus vectors. In addition, the disclosed nucleic acid sequences can be delivered to a target cell in a non-nucleic acid based system. For example, the disclosed polynucleotides can be delivered through electroporation, or through lipofection, or through calcium phosphate precipitation. The delivery mechanism chosen will depend in part on the type of cell targeted and whether the delivery is occurring for example in vivo or in vitro.

In some aspects, a vector for use in the methods disclosed can further comprise a targeting moiety. In some aspects, a vector can comprise a nucleic acid sequence capable of encoding a targeting moiety. Any one or more of the known targeting moieties can be used. For example, the targeting moiety can direct the vector to a specific cell type such as a cancer cell. In some aspects, one or more targeting moieties can be, but are not limited to, a nucleic acid sequence, a peptide, antibody, antibody fragment, or a therapeutic agent.

### 2. Nucleic Acid Sequence and Sequence of Interest

The disclosed vectors for use in the methods disclosed comprise a nucleic acid sequence. In some aspects, the nucleic acid sequence is capable of encoding a sequence of interest.

In some aspects, the nucleic acid sequence is a gene of interest. The term "gene of interest" can mean a nucleic acid sequence (e.g., a therapeutic gene), that is partly or entirely heterologous, i.e., foreign, to a cell into which it is introduced.

The term "gene of interest" can also mean a nucleic acid sequence that is partly or entirely complementary to an endogenous gene of the cell into which it is introduced. In some aspects, the term "gene of interest" does not comprise a complete gene sequence.

In some aspects, a nucleic acid sequence can be a nucleic acid sequence that has therapeutic properties itself. For example, the nucleic acid sequence can be micro RNA, shRNA, or siRNA, wherein the micro RNA, shRNA or siRNA is a therapeutic agent.

In some aspects, a nucleic acid sequence in the disclosed vectors for use in the methods disclosed herein can also include one or more transcriptional regulatory sequences and any other nucleic acid, such as introns, that may be necessary for optimal expression of a selected nucleic acid.

In some aspects, a nucleic acid sequence or gene of interest in the disclosed vectors for use in the methods disclosed herein is capable of encoding a sequence of interest. A "sequence of interest" means a peptide or polypeptide sequence (e.g., a therapeutic protein), that is expressed from a nucleic acid sequence.

In some aspects, the sequence of interest is a selectable marker, detectable marker, or a therapeutic agent. In some aspects, the disclosed vectors comprise a selectable marker or detectable marker that is separate from the nucleic acid sequence capable of encoding a sequence of interest or from the nucleic acid sequence that is the therapeutic itself.

As stated above when discussing the vectors, examples of suitable selectable markers can be, but are not limited to, dihydrofolate reductase (DHFR), thymidine kinase, neomycin, neomycin analog G418, hydromycin, puromycin, hygromycin resistance, neomycin resistance or β-galactosidase (β-gal). The selectable marker can be present in the vector backbone or be the sequence of interest.

Detectable markers can be used for the purpose of tracing or visualizing the location of the vector expressing the detectable marker. Exemplary detectable markers include, but are not limited to: various fluorescent entities such as green fluorescent protein (GFP), blue fluorescent protein, cyan fluorescent protein, red fluorescent protein, and various derivatives thereof; other fluorescent proteins such as (but not limited to) dsRed, eqFP611, Dronpa, TagRFPs, KFP, EosFP, Dendra, IrisFP; other similar molecules known in the art including, but not limited to, quantum dots and other fluorescent dyes; and any derivatives or combinations thereof. The detectable marker can be present in the vector backbone or be the sequence of interest. In some aspects, a detectable marker can also be a selectable marker.

In some aspects, a therapeutic agent can be, but is not limited to, cDNA, DNA, mRNA, micro RNA, shRNA, siRNA, ribozyme, a guide RNA molecule (gRNA), an RNA-guided endonuclease protein or a Cas9 protein or a Cpf1 protein CRISPR-Cas. In some aspects, the therapeutic agent can be a chemotherapeutic agent, an anti-viral agent, an antibacterial agent, an immunosuppressant, or an immune activator.

In some aspects, a chemotherapeutic agent can be, but is not limited to, an alkylating agent, an antimetabolite agent, an antineoplastic antibiotic agent, and a mitotic inhibitor agent.

In a further aspect, the antineoplastic antibiotic agent is selected from doxorubicin, mitoxantrone, bleomycin, daunorubicin, dactinomycin, epirubicin, idarubicin, plicamycin, mitomycin, pentostatin, and valrubicin, or a pharmaceutically acceptable salt thereof.

In a further aspect, the antimetabolite agent is selected from gemcitabine, 5-fluorouracil, capecitabine, hydroxyurea, mercaptopurine, pemetrexed, fludarabine, nelarabine, cladribine, clofarabine, cytarabine, decitabine, pralatrexate, floxuridine, methotrexate, and thioguanine, or a pharmaceutically acceptable salt thereof.

In a further aspect, the alkylating agent is selected from carboplatin, cisplatin, cyclophosphamide, chlorambucil, melphalan, carmustine, busulfan, lomustine, dacarbazine, oxaliplatin, ifosfamide, mechlorethamine, temozolomide, thiotepa, bendamustine, and streptozocin, or a pharmaceutically acceptable salt thereof.

In a further aspect, the mitotic inhibitor agent is selected from irinotecan, topotecan, rubitecan, cabazitaxel, docetaxel, paclitaxel, etopside, vincristine, ixabepilone, vinorelbine, vinblastine, and teniposide, or a pharmaceutically acceptable salt thereof.

In some aspects, the disclosed vectors can comprise two nucleic acid sequences, wherein one nucleic acid sequence is capable of encoding a sequence of interest, wherein the sequence of interest is a selectable or detectable marker and a second nucleic acid sequence is capable of encoding a sequence of interest, wherein the sequence of interest is a therapeutic agent. Thus, in some aspects, the disclosed vectors can comprise two sequences of interest, wherein one sequence of interest is a selectable or detectable marker and a second sequence of interest is a therapeutic agent.

In some aspects, the disclosed vectors can comprise two nucleic acid sequences, wherein one nucleic acid sequence is capable of encoding a sequence of interest, wherein the sequence of interest is a selectable or detectable marker and a second nucleic acid sequence is a therapeutic agent. Thus, in some aspects one of the two nucleic acid sequences does not encode a therapeutic agent but instead the nucleic acid sequence is the therapeutic agent.

### 3. Pharmaceutical Compositions and Delivery

Disclosed herein are pharmaceutical compositions comprising one or more of the vectors described herein. In some aspects, the vectors described herein can be provided in a pharmaceutical composition. For example, the vectors described herein can be formulated with a pharmaceutically acceptable carrier.

In some aspects, a pharmaceutical composition can comprise a therapeutic agent. In some aspects, a pharmaceutical composition can comprise a therapeutic agent and one or more of the vectors described herein. For example, disclosed herein are pharmaceutical compositions comprising one or more of the vectors described herein and a therapeutic such as a chemotherapeutic. In some aspects, the chemotherapeutic can be, but is not limited to, an anticancer drug, a cytotoxic drug, pain-management drug, pseudomonas exotoxin A, a non-radioactive isotope (e.g. boron-10 for boron neutron capture therapy), and/or a photosensitizer (e.g. photofrin, foscan, 5-aminolevulinic acid, Mono-L-aspartyl chlorin e6, pthalocyanines, Meta-tetra(hydroxyphenyl)porphyrins,texaphyrins, Tin ethyl etipurpurin). In some aspects, a chemotherapeutic agent can be, but is not limited to, an alkylating agent, an antimetabolite agent, an antineoplastic antibiotic agent, a mitotic inhibitor agent, a checkpoint inhibitor, and a kinase inhibitor. Any of the therapeutic agents listed above as examples of a sequence of interest can also be provided separate from the vector as part of the pharmaceutical composition.

Disclosed herein are compositions comprising one or more of the vectors described herein that that further comprise a carrier such as a pharmaceutically acceptable carrier. For example, disclosed are pharmaceutical compositions, comprising the vectors disclosed herein, and a pharmaceutically acceptable carrier.

For example, pharmaceutical compositions comprising the vectors described herein can comprise a pharmaceutically acceptable carrier. By "pharmaceutically acceptable" is meant a material or carrier that would be selected to minimize any degradation of the active ingredient and to minimize any adverse side effects in the subject, as would be well known to one of skill in the art. Examples of carriers include dimyristoylphosphatidyl (DMPC), phosphate buffered saline or a multivesicular liposome. For example, PG:PC:Cholesterol:peptide or PC:peptide can be used as carriers in this disclosure. Other suitable pharmaceutically acceptable carriers and their formulations are described in Remington: The Science and Practice of Pharmacy (19th ed.) ed. A.R. Gennaro, Mack Publishing Company, Easton, PA 1995. Typically, an appropriate amount of pharmaceutically-acceptable salt is used in the formulation to render the formulation isotonic. Other examples of the pharmaceutically-acceptable carrier include, but are not limited to, saline, Ringer's solution and dextrose solution. The pH of the solution can be from about 5 to about 8, or from about 7 to about 7.5. Further carriers include sustained release preparations such as semi-permeable matrices of solid hydrophobic polymers containing the composition, which matrices are in the form of shaped articles, e.g., films, stents (which are implanted in vessels during an angioplasty procedure), liposomes or microparticles. It will be apparent to those persons skilled in the art that certain carriers may be more preferable depending upon, for instance, the route of administration and concentration of vector being administered. These most typically would be standard carriers for administration of drugs to humans, including solutions such as sterile water, saline, and buffered solutions at physiological pH.

Pharmaceutical compositions can also include carriers, thickeners, diluents, buffers, preservatives and the like, as long as the intended activity of the vector of the present invention is not compromised. Pharmaceutical compositions may also include one or more active ingredients (in addition to the vector of the present invention) such as antimicrobial agents, anti-inflammatory agents, anesthetics, and the like. The pharmaceutical composition may be administered in a number of ways depending on whether local or systemic treatment is desired, and on the area to be treated.

Preparations of parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Formulations for optical administration may include ointments, lotions, creams, gels, drops, suppositories, sprays, liquids and powders. Conventional pharmaceutical carriers, aqueous, powder or oily bases, thickeners and the like may be necessary or desirable.

Compositions for oral administration include powders or granules, suspensions or solutions in water or non-aqueous media, capsules, sachets, or tablets. Thickeners, flavorings, diluents, emulsifiers, dispersing aids, or binders may be desirable. Some of the compositions may potentially be administered as a pharmaceutically acceptable acid- or base-addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mon-, di-, trialkyl and aryl amines and substituted ethanolamines.

In the methods described herein, delivery (or administration or introduction) of the vector or pharmaceutical compositions disclosed herein to subjects can be via a variety of mechanisms.

The disclosed methods comprise introducing a vector to a cell. In some aspects, the methods comprising introducing a vector to a target site. In some aspects the methods comprise introducing a vector to a subject. Thus, a subject comprises a cell that is the target site for the disclosed vectors. In some aspects, all of the disclosed methods comprising introducing a vector to a cell also comprise introducing a vector to a subject.

### C. Alternating Electric Fields

The methods disclosed herein comprise alternating electric fields. In some aspects, the alternating electric field used in the methods disclosed herein is a tumor-treating field. In some aspects, the alternating electric field can vary dependent on the type of cell or condition to which the alternating electric field is applied. In some aspects, the alternating electric field can be applied through one or more electrodes placed on the subject's body. In some aspects, there can be two or more pairs of electrodes. For example, arrays can be placed on the front/back and sides of a patient and can be used with the systems and methods disclosed herein. In some aspects, where two pairs of electrodes are used, the alternating electric field can alternate between the pairs of electrodes. For example, a first pair of electrodes can be placed on the front and back of the subject and a second pair of electrodes can be placed on either side of the subject, the alternating electric field can then be applied and can alternate between the front and back electrodes and then to the side to side electrodes.

In some aspects, the frequency of the alternating electric field is between 50 kHz and 1 MHz. In some aspects, the frequency of the alternating electric field is between 100 and 500 kHz. The frequency of the alternating electric fields can also be, but is not limited to, between 50 and 500 kHz, between 100 and 500 kHz, between 25 kHz and 1 MHz, between 50 and 190 kHz, between 25 and 190 kHz, between 180 and 220 kHz, or between 210 and 400 kHz. In some aspects, the frequency of the alternating electric fields can be electric fields at 50 kHz, 100 kHz, 150 kHz, 200 kHz, 250 kHz, 300 kHz, 350 kHz, 400 kHz, 450 kHz, 500 kHz, 600 kHz, 700 kHz, 800 kHz, 900 kHz, 1 MHz, or any frequency between. In some aspects, the frequency of the alternating electric field is from about 200 kHz to about 400 kHz, from about 250 kHz to about 350 kHz, and may be around 300 kHz.

In some aspects, the field strength of the alternating electric fields can be between 1 and 4 V/cm RMS. In some aspects, different field strengths can be used (e.g., between 0.1 and 10 V/cm). In some aspects, the field strength can be 1.75 V/cm RMS. In some embodiments the field strength is at least 1 V/cm RMS. In other embodiments, combinations of field strengths are applied, for example combining two or more frequencies at the same time, and/or applying two or more frequencies at different times.

In some aspects, the alternating electric fields can be applied for a variety of different intervals ranging from 0.5 hours to 72 hours. In some aspects, a different duration can be used (e.g., between 0.5 hours and 14 days). In some aspects, application of the alternating electric fields can be repeated periodically. For example, the alternating electric fields can be applied every day for a two hour duration.

In some aspects, the exposure may last for at least 6 hours, at least 12 hours, at least 24 hours, at least 36 hours, at least 48 hours, or at least 72 hours or more.

The disclosed methods comprise applying one or more alternating electric fields to a cell or to a subject. In some aspects, the alternating electric field is applied to a target site or tumor target site. When applying alternating electric fields to a cell, this can often refer to applying alternating electric fields to a subject comprising a cell. Thus, applying alternating electric fields to a target site of a subject results in applying alternating electric fields to a cell.

### D. Methods of Transporting

Disclosed are methods for transporting a composition across a cell membrane of a cell, the method comprising applying an alternating electric field to the cell for a period of time, wherein application of the alternating electric field increases permeability of the cell membrane; and introducing the composition to the cell, wherein the increased permeability of the cell membrane enables the composition to cross the cell membrane. In some aspects, the composition is a vector, comprises a vector, or is a pharmaceutical composition comprising a vector.

In some aspects, any of the vectors or pharmaceutical compositions disclosed herein can be used in the disclosed methods for transporting a composition (e.g. a vector) across a cell membrane of a cell.

Disclosed are methods for transporting a vector across a cell membrane of a cell, the method comprising applying an alternating electric field to the cell for a period of time, wherein application of the alternating electric field increases permeability of the cell membrane; and introducing the vector to the cell, wherein the increased permeability of the cell membrane enables the vector to cross the cell membrane.

Also disclosed are methods for improving the transport of a vector across a cell membrane of a cell, the method comprising applying an alternating electric field to the cell for a period of time, wherein application of the alternating electric field increases permeability of the cell membrane; and introducing the vector to the cell, wherein the increased permeability of the cell membrane enables the vector to cross the cell membrane.

In some aspects, the cells are target cells. In some aspects, the cells are cancer or tumor cells. In some aspects, the cells are not cancer or tumor cells. In some aspects, the cells are viral-infected cells. In some aspects, the cancer cells can be, but are not limited to, glioblastoma cells, uterine sarcoma cells, breast adenocarcinoma cells, pancreatic cancer cells, non-small cell lung cancer, hepatocellular, gastric cancer cells, or brain cancer cells. In some aspects, the cells can be any cell in need of gene therapy. For example, cells that are deficient in a particular protein can be targeted by the alternating electric fields so that a vector can be used to deliver the deficient protein to the cell. In some aspects, the cell can be a viral-infected cell, wherein the cell is infected with a virus selected from, but not limited to, influenza virus, adenovirus, coronavirus, and lentivirus.

In some aspects, the methods for transporting a composition or vector across a cell membrane of a cell comprise, in part, applying an alternating electric field to the cell for a period of time. In some aspects, an alternating electric field can be used to introduce the vector into cancer cells only. In some aspects, the alternating electric field can be applied at a frequency of about 200 kHz. In some aspects, the alternating electric field can be applied at a frequency between 50 and 190 kHz. In some aspects, the alternating electric field can be applied at a frequency between 210 and 400 kHz. In some aspects, the alternating electric field can be applied at a frequency between 250 and 350 kHz. In some aspects, the alternating electric field has a field strength of at least 1 V/cm RMS. In some aspects, the alternating electric field has a frequency between 50 and 1 MHz. In some aspects, the alternating electric field has a frequency between 50 and 190 kHz. In some aspects, the alternating electric field has a frequency between 210 and 400 kHz. In some aspects, the alternating electric field has a frequency between 250 and 350 kHz. In some aspects, the alternating electric field has a field strength of at least 1 V/cm RMS. In some aspects, the alternating electric field has a field strength between 1 and 4 V/cm RMS.

In some aspects, the step of introducing the composition or vector begins at a given time, and wherein the step of applying the alternating electric field ends at least 12 hours after the given time. In some aspects, the step of applying the alternating electric field begins at least one hour before the given time. In some aspects, the step of applying the alternating electric field begins at least one to around twenty-four hours before the given time.

In some aspects, applying the alternating electric field and introducing the composition or vector occur simultaneously. In some aspects, applying the alternating electric field and introducing the composition or vector occur consecutively. In some aspects, applying the alternating electric field occurs prior to introducing the composition or vector.

In some aspect, the cell is a cancer cell, wherein the cancer cells comprise glioblastoma cells and the alternating electric field has a frequency is between 250 kHz and 350 kHz.

In some aspect, the cell is a cancer cell, wherein the cancer cells comprise uterine sarcoma cells, and the alternating electric field has a frequency is between 125 kHz and 175 kHz.

In some aspect, the cell is a cancer cell, wherein the cancer cells comprise breast adenocarcinoma cells, and the alternating electric field has a frequency is between 75 kHz and 175 kHz.

In some aspects, the composition or vector is introduced intratumorally, intracranially, intraventricularly, intrathecally, epidurally, intradurally, intravascularly, intravenously (targeted or non-targeted), intraarterially, intramuscularly, subcutaneously, intraperitoneally, orally, intranasally, via intratumor injection (e.g. computed tomography-guided, during surgery or biopsy) or via inhalation.

In some aspects, the composition or vector is introduced to the cell in a targeted or non-targeted manner.

In some aspects, the composition or vector is introduced once, twice, three or more times. The time between administrations can be hours, days, weeks, months or years.

In some aspects, the composition or vector comprises a nucleic acid sequence capable of encoding a sequence of interest. In some aspects, the sequence of interest is a selectable marker, detectable marker, or a therapeutic agent. In some aspects, the vector comprises a nucleic acid sequence, wherein the nucleic acid sequence is a therapeutic agent. For example, the nucleic acid sequence can be micro RNA, shRNA, or siRNA, wherein the micro RNA, shRNA or siRNA is a therapeutic agent.

In some aspects, the selectable marker or detectable marker can be, but is not limited to, dihydrofolate reductase (DHFR), thymidine kinase, neomycin, neomycin analog G418, hydromycin, puromycin, hygromycin resistance, neomycin resistance, β-galactosidase (β-gal), fluorescent protein (e.g. green, blue, red fluorescent protein).

In some aspects, the disclosed compositions or vectors can comprise two nucleic acid sequences, wherein one nucleic acid sequence is capable of encoding a sequence of interest, wherein the sequence of interest is a selectable or detectable marker and a second nucleic acid sequence is capable of encoding a sequence of interest, wherein the sequence of interest is a therapeutic agent. Thus, in some aspects, the disclosed compositions or vectors can comprise two sequences of interest, wherein one sequence of interest is a selectable or detectable marker and a second sequence of interest is a therapeutic agent.

In some aspects, the disclosed compositions or vectors can comprise two nucleic acid sequences, wherein one nucleic acid sequence is capable of encoding a sequence of interest, wherein the sequence of interest is a selectable or detectable marker and a second nucleic acid sequence is a therapeutic agent. Thus, in some aspects one of the two nucleic acid sequences does not encode a therapeutic agent but instead the nucleic acid sequence is the therapeutic agent.

In some aspects, the vector is a plasmid, cosmid, phagemid or a viral vector. In some aspects, the vector is a viral vector. For example, the viral vector can be, but is not limited to, an adenovirus, an adeno-associated virus, a lentivirus or a herpes virus. In some aspects, the vector can be any of the vectors described herein.

In some aspects, the composition or vector is provided in a pharmaceutical composition. In some aspects, the pharmaceutical composition can be one or more of the pharmaceutical compositions disclosed herein. In some aspects, the pharmaceutical composition can further comprise a therapeutic agent, such as a chemotherapeutic agent.

In some aspects, a composition or vector can further comprise a targeting moiety. In some aspects, a vector can comprise a nucleic acid sequence capable of encoding a targeting moiety. Any one or more of the known targeting moieties can be used. For example, the targeting moiety can direct the composition or vector to a specific cell type such as a cancer cell. In some aspects, one or more targeting moieties can be, but are not limited to, a nucleic acid sequence, a peptide, antibody, antibody fragment, or a therapeutic agent.

In some aspects, the methods of PCT/US19/40479 filed on July 3, 2019 can be used in the methods disclosed herein. For example, PCT/US 19/40479 filed on July 3, 2019 describes methods and processes to deliver a substance across a cell membrane of a cell. For example, PCT/US 19/40479 filed on July 3, 2019 describes methods and processes that comprise applying an alternating electric field to the cell for a period of time, wherein application of the alternating electric field increases permeability of the cell membrane; and introducing the substance to a vicinity of the cell, wherein the increased permeability of the cell membrane enables the substance to cross the cell membrane.

In some aspects, a composition can be transported across a cell membrane *in vivo* or *in vitro.* In some aspects, a composition can be administered to a subject comprising a cell and once inside the subject, the composition can be transported across a cell membrane in combination with application of alternating electric fields. In some aspects, a composition can be administered to one or more cells in cell culture as well as the application of alternating electric fields. In some aspects, once the composition has been transported across the cell membrane in combination with application of alternating electric fields, the cell can be introduced or reintroduced into a subject.

### E. Methods of Treating

The above methods of transporting a vector into a cell can be used for treating a subject in need thereof. In some aspects, the use of alternating electric fields allow for or increase transport of a composition or vector across a cell membrane wherein once inside the cell, the composition or vector can provide a therapeutic, thus treating a subject in need thereof.

The disclosed methods of treating can include the application of alternating electric fields in vivo or in vitro. Thus, in some aspects, applying an alternating electric field to a cell comprises applying an alternating electric field to a subject comprising a cell. In some aspects, applying an alternating electric field to a cell comprises applying an alternating electric field to a cell in culture and then the cell can be introduced into the subject being treated. In some aspects, once the composition has been transported across the cell membrane in combination with application of alternating electric fields, the cell can be introduced or reintroduced into a subject.

In some aspects, an additional therapeutic or therapeutic regimen (e.g. chemotherapy or radiotherapy) can be administered to the subject prior to, contemporaneous with or after the composition (e.g. vector) is transported into a cell of the subject.

### 1. Treating with vector

Disclosed are methods for treating a subject in need thereof, the method comprising applying an alternating electric field to the cell for a period of time, wherein application of the alternating electric field increases permeability of the cell membrane; and introducing the composition to the cell, wherein the increased permeability of the cell membrane enables the composition to cross the cell membrane, wherein once inside the cell, the composition provides a therapeutic effect to the subject. In some aspects, the composition is a vector or pharmaceutical composition comprising a vector.

In some aspects, any of the vectors or pharmaceutical compositions disclosed herein can be used in the disclosed methods for transporting a vector across a cell membrane of a cell.

Disclosed are methods for treating a subject in need thereof, the method comprising applying an alternating electric field to the cell for a period of time, wherein application of the alternating electric field increases permeability of the cell membrane; and introducing the vector to the cell, wherein the increased permeability of the cell membrane enables the vector to cross the cell membrane, wherein once inside the cell, the vector provides a therapeutic effect to the subject.

In some aspects, a subject in need thereof can be a subject with any diagnosed medical condition that can be treated with a vector carrying a therapeutic. For example, a subject that could benefit from gene therapy would be a subject in need thereof. In some aspects, a subject in need of having one or more genes knocked out or knocked down would be a subject in need thereof. In some aspects, the subject can be a subject diagnosed with cancer. In some aspects, the subject can be a subject diagnosed with an autoimmune disorder. In some aspects, the subject can be a subject diagnosed with a protein deficiency.

In some aspects, a therapeutic agent can be, but is not limited to, cDNA, DNA, mRNA, micro RNA, shRNA, siRNA, ribozyme, a guide RNA molecule (gRNA), an RNA-guided endonuclease protein or a Cas9 protein or a Cpf1 protein CRISPR-Cas. In some aspects, the therapeutic agent can be a chemotherapeutic agent, an anti-viral agent, an antibacterial agent, an immunosuppressant, or an immune activator.

In some aspects, a therapeutic agent is a specific protein that a subject may be deficient in. In some aspects, a therapeutic agent can be one or more elements of the CRISPR-Cas9 system. For example, in some aspects, a therapeutic agent can be a gRNA that targets a specific gene to be cleaved or an endonuclease that cleaves at a gRNA target site. In one example, a vector can comprise a gRNA that targets PD-1. Using CRISPR to reduce or eliminate PD-1 expression on T cells can allow for better killing of cancer cells. In some aspects, PD-1 on T cells would bind to PD-L1 on cancer cells signaling to the T cell that the cell is a healthy cell and therefore the T cell does not kill the cancer cell. Using the disclosed methods to introduce a vector comprising a gRNA targeting PD-1 would aide in the process of cleaving the PD-1 gene and reducing expression thus resulting in more killing of cancer cells expressing PD-L1.

In some aspects, an additional therapeutic or therapeutic regimen (e.g. chemotherapy or radiotherapy) can be administered to the subject prior to, contemporaneous with or after the composition (e.g. vector) is transported into a cell of the subject.

### 2. Treating with vector plus second alternating electric field

Disclosed are methods for treating a subject in need thereof, the method comprising applying a first alternating electric field at a first frequency to the cell for a first period of time, wherein application of the first alternating electric field at the first frequency to the cell for the first period of time increases permeability of cell membrane of the cell; introducing a vector to the cell, wherein the increased permeability of the cell membrane of the cell enables the vector to cross the cell membrane, wherein the vector comprises a nucleic acid sequence capable of encoding a therapeutic agent; and applying a second alternating electric field at a second frequency to the cell for a second period of time, wherein the second frequency is different from the first frequency.

In some aspects, the cell can be a viral-infected cell. In some aspects, the vector comprises a nucleic acid sequence that is a therapeutic agent or that is capable of encoding a therapeutic agent. For example, the vector can comprises a nucleic acid sequence that is capable of encoding an anti-viral agent or an immune modulator.

In some aspects, the second alternating electric field prevents viral replication and/or infection. Therefore, the treatment is a dual treatment of a therapeutic agent provided by the vector and the alternating electric field that inhibits viral replication and/or infection.

In some aspects, the first and second alternating electrical fields are different. The first and second alternating electrical fields can be one or more of those disclosed herein. In some aspects, the first or second alternating electrical fields, or both, can have a frequency between 50 kHz and 1 MHz. The alternating electrical fields can vary based on the cell type. For example, the first alternating electrical field can have a first frequency between 250 kHz and 350 kHz and the second alternating electrical field can have a frequency between 150 kHz and 250 kHz; the first alternating electrical field can have a first frequency between 125 kHz and 175 kHz and the second alternating electrical field can have a frequency between 75 kHz and 125 kHz; or the first alternating electrical field can have a first frequency between 75 kHz and 175 kHz and the second alternating electrical field can have a frequency between 100 kHz and 300 kHz. In some aspects, the alternating electric field has a field strength of at least 1 V/cm RMS. In some aspects, the alternating electric field has a field strength between 1 and 4 V/cm RMS

In some aspects, the second period of time comprises a plurality of non-contiguous intervals of time during which the second alternating electric field at the second frequency is applied to the cells, wherein the plurality of non-contiguous intervals of time collectively add up to at least one week.

In some aspects, the first alternating electric field and second alternating electric field can both occur in vivo. In some aspects, the first alternating electric field and second alternating electric field can both occur *in vitro.* In some aspects, the first alternating electric field occurs *in vitro* and the second alternating electric field occurs *in vivo.* In some aspects, once the composition has been transported across the cell membrane in combination with application of alternating electric fields, the cell can be introduced or reintroduced into a subject.

In some aspects, an additional therapeutic or therapeutic regimen (e.g. chemotherapy or radiotherapy) can be administered to the subject prior to, contemporaneous with or after the composition (e.g. vector) is transported into a cell of the subject.

### F. Methods of Killing a Cell

The above methods of transporting a vector into a cell can be used for killing a cell. In some aspects, the use of alternating electric fields allow for or increase transport of a vector across a cell membrane wherein once inside the cell, the vector can provide a therapeutic, wherein the therapeutic results in cell death.

The above methods of transporting a vector into a cell can be used for inducing apoptosis of a cell. In some aspects, the use of alternating electric fields allow for or increase transport of a vector across a cell membrane wherein once inside the cell, the vector can provide a therapeutic, wherein the therapeutic results in apoptosis of the cell.

In some aspects, an additional therapeutic or therapeutic regimen (e.g. chemotherapy or radiotherapy) can be administered to the subject prior to, contemporaneous with or after the composition (e.g. vector) is transported into a cell of the subject.

### 1. Reducing Viability of a Cell with vector therapeutic

Disclosed are methods of reducing the viability of a cell comprising applying a first alternating electric field at a first frequency to the cell for a period of time, wherein application of the first alternating electric field increases permeability of the cell membrane; introducing a vector to the cell, wherein the increased permeability of the cell membrane enables the vector to cross the cell membrane, wherein the vector comprises a nucleic acid sequence capable of encoding a therapeutic agent and wherein, once the vector crosses the cell membrane, the vector encodes the therapeutic agent, wherein the presence of the therapeutic agent reduces the viability of the cell. In some aspects, the cell is a tumor or cancer cell. In some aspects, the cancer cells are glioblastoma cells, uterine sarcoma cells, breast adenocarcinoma cells, pancreatic cancer cells, non-small cell lung cancer, hepatocellular, gastric cancer cells, or brain cancer cells.

In some aspects, the therapeutic agent can be a pro-apoptotic factor. In some aspects, the therapeutic can be, but is not limited to, a cytokine, p53, a suicide gene (e.g. cytosine deaminase/5-fluorocytosine and the herpes simplex virus/ganciclovir), or an activator of known apoptosis genes.

In some aspects, the alternating electric fields can be any of those described for transporting a vector into a cell.

In some aspects, the cell is a tumor or cancer cell. In some aspects, the cancer cells are glioblastoma cells, uterine sarcoma cells, breast adenocarcinoma cells, pancreatic cancer cells, non-small cell lung cancer, hepatocellular, gastric cancer cells, or brain cancer cells.

In some aspects, the step of introducing the vector begins at a given time, and wherein the step of applying the alternating electric field ends at least 12 hours after the given time. In some aspects, the step of applying the alternating electric field begins at least one hour before the given time. In some aspects, the step of applying the alternating electric field begins at least one to around twenty-four hours before the given time.

In some aspects, an additional therapeutic or therapeutic regimen (e.g. chemotherapy or radiotherapy) can be administered to the subject prior to, contemporaneous with or after the composition (e.g. vector) is transported into a cell of the subject.

### 2. Reducing Viability of a Cell with vector therapeutic plus second alternating electric field

In some aspects, the disclosed methods of reducing the viability of a cell can further comprise administering a second alternating electrical field at a second frequency.

Disclosed are methods for reducing the viability of a cell, the method comprising: applying a first alternating electric field at a first frequency to the cell for a first period of time, wherein application of the first alternating electric field at the first frequency to the cell for the first period of time increases permeability of cell membrane of the cancer cell; introducing a vector to the cell, wherein the increased permeability of the cell membrane of the cell enables the vector to cross the cell membrane; and applying a second alternating electric field at a second frequency to the cell for a second period of time, wherein the second frequency is different from the first frequency, and wherein the second alternating electric field at the second frequency reduces viability of the cell.

Thus, in some aspects, the second alternating electric field is a tumor-treating field. In some aspects, the first and second alternating electrical fields are different. The first and second alternating electrical fields can be one or more of those disclosed herein. In some aspects, the first or second alternating electrical fields, or both, can have a frequency between 50 kHz and 1 MHz. For example, in some aspects, the cancer cells comprise glioblastoma cells and the first alternating electrical field can have a first frequency between 250 kHz and 350 kHz and the second alternating electrical field can have a frequency between 150 kHz and 250 kHz. In some aspects, the cancer cells comprise uterine sarcoma cells and the first alternating electrical field can have a first frequency between 125 kHz and 175 kHz and the second alternating electrical field can have a frequency between 75 kHz and 125 kHz. In some aspects, the cancer cells comprise breast adenocarcinoma cells and the first alternating electrical field can have a first frequency between 75 kHz and 175 kHz and the second alternating electrical field can have a frequency between 100 kHz and 300 kHz. The alternating electrical fields can vary based on the cell type.

In some aspects, the first alternating electric field has a field strength of at least 1 V/cm RMS.

In some aspects, the second alternating electric field is the treatment. In some aspects, a second alternating electric field can kill a cell, such as a cancer cell.

The disclosed methods of reducing the viability of a cell a cell by using alternating electric fields to transport a vector inside a cell can be combined with the use of alternating electric fields for a more efficient response (increased reduction of viability of the cell or increased apoptosis).

Disclosed are methods for reducing the viability of a cell, the method comprising: applying a first alternating electric field at a first frequency to the cell for a first period of time, wherein application of the first alternating electric field at the first frequency to the cell for the first period of time increases permeability of the cell membranes of the cell; introducing a vector to the cell, wherein the increased permeability of the cell membranes enables the vector to cross the cell membrane; and applying a second alternating electric field at a second frequency to the cell for a second period of time, wherein the second frequency is different from the first frequency, and wherein the second alternating electric field at the second frequency reduces viability of the cell.

In some aspects, the cell is a tumor or cancer cell. In some aspects, the cancer cells are glioblastoma cells, uterine sarcoma cells, breast adenocarcinoma cells, pancreatic cancer cells, non-small cell lung cancer, hepatocellular, gastric cancer cells, or brain cancer cells.

In some aspects, the second alternating electric field is a tumor-treating field. In some aspects, the first and second alternating electrical fields are different. The first and second alternating electrical fields can be one or more of those disclosed herein.. In some aspects, the first or second alternating electrical fields, or both, can have a frequency between 50 kHz and 1 MHz. The alternating electrical fields can vary based on the cell type. For example, regardless of cell type, the first alternating electrical field can have a first frequency between 250 kHz and 350 kHz and the second alternating electrical field can have a frequency between 150 kHz and 250 kHz; the first alternating electrical field can have a first frequency between 125 kHz and 175 kHz and the second alternating electrical field can have a frequency between 75 kHz and 125 kHz; or the first alternating electrical field can have a first frequency between 75 kHz and 175 kHz and the second alternating electrical field can have a frequency between 100 kHz and 300 kHz. In some aspects, the alternating electric field has a field strength of at least 1 V/cm RMS. In some aspects, the alternating electric field has a field strength between 1 and 4 V/cm RMS.

In some aspects, the step of introducing the vector begins at a given time, and wherein the step of applying the alternating electric field ends at least 12 hours after the given time. In some aspects, the step of applying the alternating electric field begins at least one hour before the given time. In some aspects, the step of applying the alternating electric field begins at least one to around twenty-four hours before the given time.

In some aspects, the second period of time comprises a plurality of non-contiguous intervals of time during which the second alternating electric field at the second frequency is applied to the cells, wherein the plurality of non-contiguous intervals of time collectively add up to at least one week.

In some aspects, the cells are present in a body of a living subject, wherein the first alternating electric field is applied to the cells by applying a first alternating electric field to the subject's body, the second alternating electric field is applied to the cells by applying a second alternating electric field to the subject's body, and wherein the introducing comprises administering the vector to the subject.

In some aspects, an additional therapeutic or therapeutic regimen (e.g. chemotherapy or radiotherapy) can be administered to the subject prior to, contemporaneous with or after the composition (e.g. vector) is transported into a cell of the subject.

### G. Methods of Detecting

### 1. Detecting

The above methods of transporting a vector into a cell can be used for detecting a cell. In some aspects, the use of alternating electric fields allow for or increase transport of a vector across a cell membrane wherein once inside the cell, the vector can encode a detectable marker, wherein the detectable marker allows for detection of the cell.

Disclosed are methods of detecting a specific cell type comprising applying a first alternating electric field at a first frequency to one or more cells comprising the specific cell type for a period of time, wherein application of the first alternating electric field increases permeability of the cell membrane; introducing a vector to the one or more cells, wherein the increased permeability of the one or more cell membranes enables the vector to cross the one or more cell membranes, wherein the vector comprises a nucleic acid sequence capable of encoding a detectable or selectable marker and wherein, once the vector crosses the one or more cell membranes, the vector encodes the detectable or selectable marker; and detecting the detectable or selectable marker, wherein the presence of the detectable or selectable marker indicates the presence of one or more of the specific cell.

In some aspects, the cell is a tumor or cancer cell. In some aspects, the cancer cells are glioblastoma cells, uterine sarcoma cells, breast adenocarcinoma cells, pancreatic cancer cells, non-small cell lung cancer, hepatocellular, gastric cancer cells, or brain cancer cells.

In some aspects, the specific cell type is a diseased cell. For example, in some aspects the specific cell type is a cancer cell. In some aspects, a diseased cell can be identified as a cell expressing a specific cell surface marker not present on healthy cells. Thus, in some aspects, the vector is targeted to a specific cell type. Adding targeting molecules into vectors is known in the art, for example, molecules that target specific ligands can be incorporated into viral surface and/or altering the viral envelope proteins. In some aspects, a cancer specific antibody (e.g. CD20 antibody) can be incorporated into a viral vector so that the CD20 antibody directs the vector to CD20 positive cells allowing the virus to transduce the CD20 positive cell. Once the virus has entered the cell, the detectable marker can be detected thus identifying the cell as a CD20 positive cell.

In some aspects, the alternating electric fields can be any of those described for transporting a vector into a cell.

In some aspects, the step of introducing the vector begins at a given time, and wherein the step of applying the alternating electric field ends at least 12 hours after the given time. In some aspects, the step of applying the alternating electric field begins at least one hour before the given time. In some aspects, the step of applying the alternating electric field begins at least one to around twenty-four hours before the given time.

### 2. Detecting and Treating

In some aspects, the methods of detecting a specific cell type can further comprise treating the cell. In some aspects, the vector further comprises a therapeutic agent. In some aspects, the therapeutic agent can be "turned on" in terms of the nucleic acid sequence capable of encoding the therapeutic agent can be activatable. In some aspects, once a specific cell type is detected (e.g. cancer cells), a therapeutic can then be administered. Thus, the therapeutic can be administered as part of the vector or separately once detection has been confirmed. In some aspects, administering a therapeutic can be performed using any of the techniques in which the vector is administered.

Disclosed are methods of detecting and treating a cell in need thereof comprising applying a first alternating electric field at a first frequency to a cell for a period of time, wherein application of the alternating electric field increases permeability of the cell membrane; introducing a vector to the cell, wherein the increased permeability of the cell membrane enables the vector to cross the cell membrane, wherein the vector comprises nucleic acid sequences capable of encoding a therapeutic agent and a detectable/selectable marker and wherein, once the vector enters the cell the vector encodes the therapeutic agent and the detectable/selectable marker; and detecting the detectable/selectable marker, wherein the presence of the detectable/selectable marker indicates the presence of a cell in need of treatment and wherein the therapeutic agent encoded by the vector treats the cell. In some aspects "treats the cell" can mean induces apoptosis of the cell.

In some aspects, the cell is a tumor or cancer cell. In some aspects, the cancer cells are glioblastoma cells, uterine sarcoma cells, breast adenocarcinoma cells, pancreatic cancer cells, non-small cell lung cancer, hepatocellular, gastric cancer cells, or brain cancer cells.

In some aspects, the disclosed methods of detecting a cell can further comprise administering a second alternating electrical field at a second frequency. Thus, in some aspects, the second alternating electric field is a tumor-treating field. In some aspects, the first and second alternating electrical fields are different. In some aspects, the first or second alternating electrical fields, or both, can have a frequency between 50 kHz and 1 MHz. The first and second alternating electrical fields can be one or more of those disclosed herein. For example, in some aspects, the cancer cells comprise glioblastoma cells and the first alternating electrical field can have a first frequency between 250 kHz and 350 kHz and the second alternating electrical field can have a frequency between 150 kHz and 250 kHz. In some aspects, the cancer cells comprise uterine sarcoma cells and the first alternating electrical field can have a first frequency between 125 kHz and 175 kHz and the second alternating electrical field can have a frequency between 75 kHz and 125 kHz. In some aspects, the cancer cells comprise breast adenocarcinoma cells and the first alternating electrical field can have a first frequency between 75 kHz and 175 kHz and the second alternating electrical field can have a frequency between 100 kHz and 300 kHz. The alternating electrical fields can vary based on the cell type. In some aspects, the alternating electric field has a field strength of at least 1 V/cm RMS. In some aspects, the alternating electric field has a field strength between 1 and 4 V/cm RMS.

In some aspects, the second alternating electric field is the treatment. In some aspects, a second alternating electric field can kill a cell, such as a cancer cell.

In some aspects, the step of introducing the vector begins at a given time, and wherein the step of applying the alternating electric field ends at least 12 hours after the given time. In some aspects, the step of applying the alternating electric field begins at least one hour before the given time. In some aspects, the step of applying the alternating electric field begins at least one to around twenty-four hours before the given time.

In some aspects, the cells are present in a body of a living subject, wherein the first alternating electric field is applied to the cells by applying a first alternating electric field to the subject's body, the second alternating electric field is applied to the cells by applying a second alternating electric field to the subject's body, and wherein the introducing comprises administering the vector to the subject.

In some aspects, an additional therapeutic or therapeutic regimen (e.g. chemotherapy or radiotherapy) can be administered to the subject prior to, contemporaneous with or after the composition (e.g. vector) is transported into a cell of the subject.

### H. Kits

The materials described above as well as other materials can be packaged together in any suitable combination as a kit useful for performing, or aiding in the performance of, the disclosed method. It is useful if the kit components in a given kit are designed and adapted for use together in the disclosed method. For example disclosed are kits for imaging and/or treating. In some aspects, the kit can comprise one or more of the disclosed vectors. The kits also can contain equipment for applying alternating electrical fields.

Disclosed herein are kits comprising one or more of the vectors described herein in and a device capable of administering an alternating electric field. For example, disclosed herein are kits comprising one or more of the vectors described herein in and a TTFields device (e.g Optune^{®}, Novocure Ltd.).

### I. Examples

The ability to transport a virus across a cell membrane was studies using A549 (ATCC^{®} CCL-185^{™}) and Influenza (PR8) was assessed.

A549 (ATCC^{®} CCL-185^{™}) and Influenza (PR8) were used. The cells were grown in DMEM (Biological Industries, 01-055-1A) supplemented with 10% fetal bovine serum (FBS) (Biological Industries, 04-007-1A).

A549 cells were seeded on glass cover slips (22mm diameter) at a density of 1.5 × 10⁵ cells/cover slip. After a 24-hour culture, the cells were transferred into inovitro dishes containing 2ml DMEM supplemented with 2% FBS. The cells were infected with the influenza (PR8) virus at 1% or 0.01% concentration over 6 hours. Following infection the cells were washed with PBS and maintained for additional 18 hours or 42 hours in DMEM supplemented with 2% FBS. TTFields were applied either during the infection and proliferation phases. At 24 hours or 48 hours after infection, the growth media were collected, cells were trypsinized, resuspended and counted using Scepter^{™} 2.0 Cell Counter (Merck, Millipore). After resuspension the cells were washed with PBS. The supernatants and pellets were frozen at -80°C and transferred to Sheba for viral genome extraction and qRT-PCR.

TTFields (150 kHz, 1.5 V/cm RMS) were applied using the inovitro^{™} system (Novocure Ltd) with the following amendments: the inovitro plates were covered with parafilm and the media were not replaced during cell culture.

Data from one experiment. Data are presented as means ± SD. Statistical significance was analyzed by the Student's t-test.

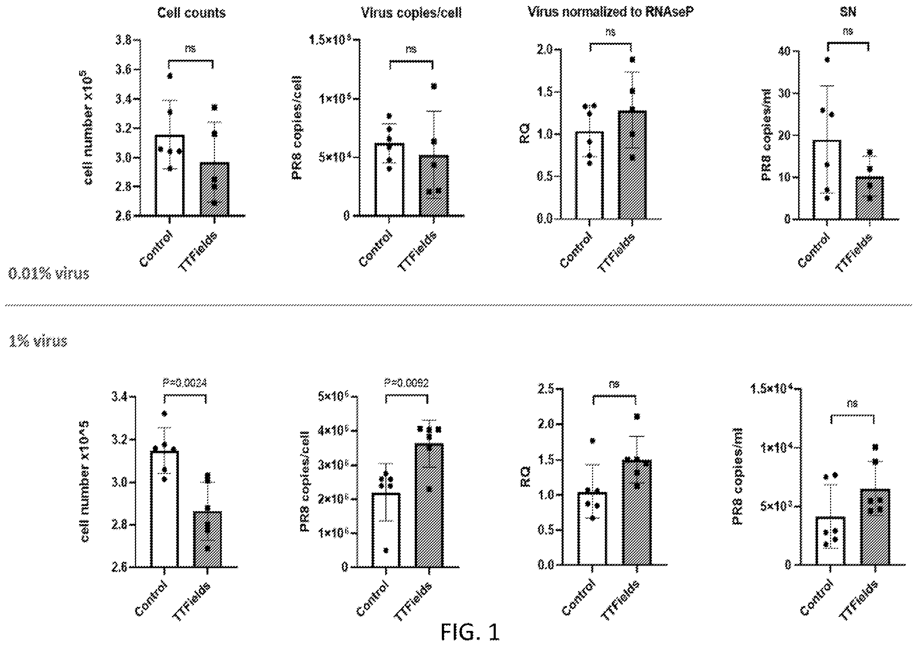

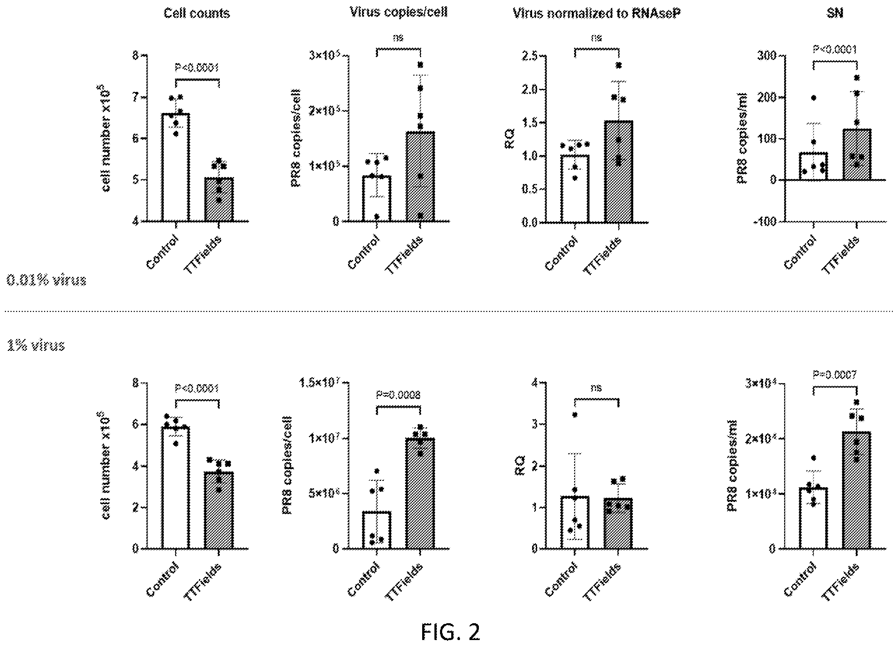

There was a decrease trend in cell number after 24 hours TTFields treatment in 0.01% virus and significant decrease in cell number in 1% virus as compared to the untreated cultures (Figure 1). After 48 hours TTFields treatment, there was significant decrease in cell number either in 0.01% either in 1% virus (Figure 2). There was an increase trend in virus copies/cell, RQ and SN after 24 hours TTFields treatment in both virus concentrations (Figure 1). After 48 hours TTFields treatment there was significant increase in virus copies in SN in both virus concentrations and in virus copies/cell in 1% virus, in addition there was an increase trend in virus copies/cell in 0.01% virus and in RQ in both concentrations (Figure 2).

Those skilled in the art will recognize, or be able to ascertain using no more than routine experimentation, many equivalents to the specific embodiments of the method and compositions described herein. The scope of the present invention is defined by the following claims.

## Claims

1. A vector for use in a method for transporting the vector across a cell membrane of a cancer cell in a subject for gene therapy, wherein the gene therapy is for detecting the cancer cell, the method comprising:
applying a first alternating electric field at a first frequency to the cell for a period of time, wherein application of the alternating electric field increases permeability of the cell membrane; and
introducing the vector to the cell, wherein the vector comprises a nucleic acid sequence capable of encoding a selectable marker and wherein, once the vector crosses the tumor or cancer cell membrane, the vector encodes the selectable marker, wherein the increased permeability of the cell membrane enables the vector to cross the cell membrane;
detecting the selectable marker, wherein the presence of the selectable marker indicates the presence of the cancer cell.

2. The vector for use according to claim 1, wherein the vector comprises two nucleic acid sequences, wherein one nucleic acid sequence is capable of encoding the selectable marker and a second nucleic acid sequence is capable of encoding a therapeutic agent or is a therapeutic agent.

3. The vector for use according to claim 2, wherein the therapeutic agent is cDNA, DNA, mRNA, micro RNA, shRNA, siRNA, ribozyme, a guide RNA molecule (gRNA), an RNA-guided endonuclease protein or a Cas9 protein or a Cpf1 protein CRISPR-Cas.

4. The vector for use according to any of claims 1 - 3, wherein the selectable marker is dihydrofolate reductase (DHFR), thymidine kinase, neomycin, neomycin analog G418, hydromycin, puromycin, green fluorescence protein (GFP), hygromycin resistance (Hyg), neomycin resistance (Neo) or β-galactosidase (β-gal).

5. The vector for use according to any of claims 1 - 4, wherein the vector is a plasmid, cosmid, phagemid or a viral vector.

6. The vector for use of claim 5, wherein the vector is a viral vector.

7. The vector for use of claim 6, wherein the viral vector is an adenovirus, an adeno-associated virus, a lentivirus or a herpes virus.

8. The vector for use according to any of claims 1 - 7, wherein the cancer cells are glioblastoma cells, uterine sarcoma cells, breast adenocarcinoma cells, pancreatic cancer cells, non-small cell lung cancer, hepatocellular, gastric cancer cells, or brain cancer cells.

9. The vector for use according to any of claims 1 - 8, wherein the alternating electric field is applied at a frequency of between:
250 kHz and 350 kHz;
50 and 190 kHz; or
210 and 400 kHz.

10. The vector for use according to any of claims 1 - 9, wherein the alternating electric field has a field strength of at least 1 V/cm RMS; or wherein the alternating electric field has a field strength between 1 and 4 V/cm RMS.

11. The vector for use of claim 8, wherein:
the cancer cells comprise glioblastoma cells and the alternating electric field has a frequency is between 250 kHz and 350 kHz; or
the cancer cells comprise uterine sarcoma cells, and the alternating electric field has a frequency is between 125 kHz and 175 kHz; or
the cancer cells comprise breast adenocarcinoma cells, and the alternating electric field has a frequency is between 75 kHz and 175 kHz.

12. The vector for use according to any of claims 1 - 11, wherein the vector is provided in a pharmaceutical composition.

13. The vector for use according to claim 12, wherein the pharmaceutical composition further comprises a chemotherapeutic agent.

14. The vector for use according to any of claims 1 - 13, wherein the vector further comprises one or more targeting moieties, optionally
wherein the one or more targeting moieties is specific for the cell, further optionally wherein the one or more targeting moieties is a peptide, antibody, antibody fragment, or a therapeutic agent.

15. The vector for use according to any preceding claim, the method further comprising administering a second alternating electrical field at a second frequency, optionally wherein the second alternating electric field is a tumor-treating field.

16. The vector for use according to any preceding claim, wherein the step of introducing the vector begins at a given time, and wherein the step of applying the first alternating electric field ends at least 12 hours after the given time.

17. The vector for use according to claim 16, wherein the step of applying the first alternating electric field begins at least one hour before the given time.

18. The vector for use according to any preceding claim, the method further comprising administering a therapeutic agent.

## Patentansprüche

1. Vektor zur Verwendung in einem Verfahren zum Transport des Vektors durch eine Zellmembran einer Krebszelle in einem Patienten im Rahmen einer Gentherapie, wobei die Gentherapie dem Nachweis der Krebszelle dient, wobei das Verfahren Folgendes umfasst:
Anlegen eines ersten elektrischen Wechselfeldes mit einer ersten Frequenz an die Zelle für einen bestimmten Zeitraum, wobei das Anlegen des elektrischen Wechselfeldes die Durchlässigkeit der Zellmembran erhöht; und
Einbringen des Vektors in die Zelle, wobei der Vektor eine Nukleinsäuresequenz umfasst, die in der Lage ist, einen selektierbaren Marker zu kodieren, und wobei der Vektor, sobald er die Membran der Tumor- oder Krebszelle passiert hat, den selektierbaren Marker kodiert, wobei die erhöhte Permeabilität der Zellmembran es dem Vektor ermöglicht, die Zellmembran zu passieren;
Nachweisen des selektierbaren Markers, wobei das Vorhandensein des selektierbaren Markers das Vorhandensein der Krebszelle anzeigt.

2. Vektor zur Verwendung gemäß Anspruch 1, wobei der Vektor zwei Nukleinsäuresequenzen umfasst, wobei eine Nukleinsäuresequenz in der Lage ist, den selektierbaren Marker zu kodieren, und eine zweite Nukleinsäuresequenz in der Lage ist, einen therapeutischen Wirkstoff zu kodieren, oder ein therapeutischer Wirkstoff ist.

3. Vektor zur Verwendung gemäß Anspruch 2, wobei das therapeutische Wirkstoff cDNA, DNA, mRNA, Mikro-RNA, shRNA, siRNA, Ribozym, ein Leit-RNA-Molekül (gRNA), ein RNA-gesteuertes Endonuklease-Protein oder ein Cas9-Protein oder ein Cpf1-Protein CRISPR-Cas ist.

4. Vektor zur Verwendung gemäß einem der Ansprüche 1 bis 3, wobei der wählbare Marker Dihydrofolatreduktase (DHFR), Thymidinkinase, Neomycin, Neomycinanalog G418, Hydromycin, Puromycin, grün fluoreszierendes Protein (GFP), Hygromycinresistenz (Hyg), Neomycinresistenz (Neo) oder β-Galactosidase (β-Gal) ist.

5. Vektor zur Verwendung gemäß einem der Ansprüche 1 bis 4, wobei der Vektor ein Plasmid, ein Cosmid, ein Phagemid oder ein viraler Vektor ist.

6. Vektor zur Verwendung gemäß Anspruch 5, wobei der Vektor ein viraler Vektor ist.

7. Vektor zur Verwendung gemäß Anspruch 6, wobei der virale Vektor ein Adenovirus, ein Adeno-assoziiertes Virus, ein Lentivirus oder ein Herpesvirus ist.

8. Vektor zur Verwendung gemäß einem der Ansprüche 1 bis 7, wobei die Krebszellen Glioblastomzellen, Uterussarkomzellen, Brustadenokarzinomzellen, Bauchspeicheldrüsenkrebszellen, nicht-kleinzellige Lungenkrebszellen, Leberkrebszellen, Magenkrebszellen oder Hirntumorzellen sind.

9. Vektor zur Verwendung gemäß einem der Ansprüche 1 bis 8, wobei das elektrische Wechselfeld mit einer Frequenz zwischen:
250 kHz und 350 kHz;
50 und 190 kHz; oder
210 und 400 kHz angelegt wird.

10. Vektor zur Verwendung gemäß einem der Ansprüche 1 bis 9, wobei das elektrische Wechselfeld eine Feldstärke von mindestens 1 V/cm RMS aufweist; oder wobei das elektrische Wechselfeld eine Feldstärke zwischen 1 und 4 V/cm RMS aufweist.

11. Vektor zur Verwendung gemäß Anspruch 8, wobei:
die Krebszellen Glioblastomzellen umfassen und das elektrische Wechselfeld eine Frequenz zwischen 250 kHz und 350 kHz aufweist; oder
die Krebszellen Uterussarkomzellen umfassen und das elektrische Wechselfeld eine Frequenz zwischen 125 kHz und 175 kHz aufweist; oder
die Krebszellen Brustadenokarzinomzellen umfassen und das elektrische Wechselfeld eine Frequenz zwischen 75 kHz und 175 kHz aufweist.

12. Vektor zur Verwendung gemäß einem der Ansprüche 1 bis 11, wobei der Vektor in einer pharmazeutischen Zusammensetzung bereitgestellt wird.

13. Vektor zur Verwendung gemäß Anspruch 12, wobei die pharmazeutische Zusammensetzung ferner ein Chemotherapeutikum umfasst.

14. Vektor zur Verwendung gemäß einem der Ansprüche 1 bis 13, wobei der Vektor ferner eine oder mehrere Zielgruppen umfasst, optional
wobei die eine oder mehreren Zielgruppen für die Zelle spezifisch sind, ferner optional
wobei die eine oder mehreren Zielgruppen ein Peptid, ein Antikörper, ein Antikörperfragment oder ein therapeutischer Wirkstoff sind.

15. Vektor zur Verwendung gemäß einem der vorhergehenden Ansprüche, wobei das Verfahren ferner das Anlegen eines zweiten elektrischen Wechselfeldes mit einer zweiten Frequenz umfasst, wobei das zweite elektrische Wechselfeld optional ein Tumorbehandlungsfeld ist.

16. Vektor zur Verwendung gemäß einem der vorhergehenden Ansprüche, wobei der Schritt des Einführens des Vektors zu einem bestimmten Zeitpunkt beginnt und wobei der Schritt des Anlegens des ersten elektrischen Wechselfeldes mindestens 12 Stunden nach dem bestimmten Zeitpunkt endet.

17. Vektor zur Verwendung gemäß Anspruch 16, wobei der Schritt des Anlegens des ersten elektrischen Wechselfeldes mindestens eine Stunde vor dem gegebenen Zeitpunkt beginnt.

18. Vektor zur Verwendung gemäß einem der vorhergehenden Ansprüche, wobei das Verfahren ferner das Verabreichen eines therapeutischen Wirkstoffs umfasst.

## Revendications

1. Vecteur destiné à être utilisé dans un procédé de transport du vecteur à travers la membrane cellulaire d'une cellule cancéreuse chez un sujet pour une thérapie génique, dans lequel la thérapie génique est destinée à la détection de la cellule cancéreuse, le procédé comprenant :
l'application d'un champ électrique alternatif à une première fréquence à la cellule pendant une période de temps, dans laquelle l'application du champ électrique alternatif augmente la perméabilité de la membrane cellulaire ; et
l'introduction du vecteur dans la cellule, dans lequel le vecteur comprend une séquence d'acide nucléique capable de coder un marqueur sélectionnable et dans lequel, une fois que le vecteur traverse la membrane de la tumeur ou de la cellule cancéreuse, le vecteur code le marqueur sélectionnable, dans lequel la perméabilité accrue de la membrane cellulaire permet au vecteur de traverser la membrane cellulaire ;
la détection du marqueur sélectionnable, dans lequel la présence du marqueur sélectionnable indique la présence de la cellule cancéreuse.

2. Vecteur destiné à être utilisé selon la revendication 1, dans lequel le vecteur comprend deux séquences d'acide nucléique, dans lequel une séquence d'acide nucléique est capable de coder le marqueur sélectionnable et une deuxième séquence d'acide nucléique est capable de coder un agent thérapeutique ou est un agent thérapeutique.

3. Vecteur destiné à être utilisé selon la revendication 2, dans lequel l'agent thérapeutique est un ADNc, un ADN, un ARNm, un micro-ARN, un ARNsh, un ARNsi, un ribozyme, une molécule d'ARN guide (ARNg), une protéine endonucléase guidée par ARN ou une protéine Cas9 ou une protéine Cpf1 CRISPR-Cas.

4. Vecteur destiné à être utilisé selon l'une quelconque des revendications 1 à 3, dans lequel le marqueur sélectionnable est la dihydrofolate réductase (DHFR), la thymidine kinase, la néomycine, l'analogue de la néomycine G418, l'hydromycine, la puromycine, la protéine fluorescente verte (GFP), la résistance à l'hygromycine (Hyg), la résistance à la néomycine (Neo) ou la β -galactosidase (β-gal).

5. Vecteur destiné à être utilisé selon l'une quelconque des revendications 1 à 4, dans lequel le vecteur est un plasmide, un cosmide, un phagemide ou un vecteur viral.

6. Vecteur destiné à être utilisé selon la revendication 5, dans lequel le vecteur est un vecteur viral.

7. Vecteur destiné à être utilisé selon la revendication 6, dans lequel le vecteur viral est un adénovirus, un virus adéno-associé, un lentivirus ou un virus de l'herpès.

8. Vecteur destiné à être utilisé selon l'une quelconque des revendications 1 à 7, dans lequel les cellules cancéreuses sont des cellules de glioblastome, des cellules de sarcome utérin, des cellules d'adénocarcinome mammaire, des cellules de cancer du pancréas, des cellules de cancer du poumon non à petites cellules, des cellules de cancer hépatocellulaire, des cellules de cancer gastrique ou des cellules de cancer du cerveau.

9. Vecteur destiné à être utilisé selon l'une quelconque des revendications 1 à 8, dans lequel le champ électrique alternatif est appliqué a une fréquence parmi :
250 kHz et 350 kHz ;
50 et 190 kHz ; ou
210 et 400 kHz.

10. Vecteur destiné à être utilisé selon l'une quelconque des revendications 1 à 9, dans lequel le champ électrique alternatif a une intensité de champ d'au moins 1 V/cm RMS ; ou dans lequel le champ électrique alternatif a une intensité de champ comprise entre 1 et 4 V/cm RMS.

11. Vecteur destiné à être utilisé selon la revendication 8, dans lequel :
les cellules cancéreuses comprennent des cellules de glioblastome et le champ électrique alternatif a une fréquence comprise entre 250 kHz et 350 kHz ; ou
les cellules cancéreuses comprennent des cellules de sarcome utérin, et le champ électrique alternatif a une fréquence comprise entre 125 kHz et 175 kHz ; ou
les cellules cancéreuses comprennent des cellules d'adénocarcinome mammaire, et le champ électrique alternatif a une fréquence comprise entre 75 kHz et 175 kHz.

12. Vecteur destiné à être utilisé selon l'une quelconque des revendications 1 à 11, dans lequel le vecteur est fourni dans une composition pharmaceutique.

13. Vecteur destiné à être utilisé selon la revendication 12, dans lequel la composition pharmaceutique comprend en outre un agent chimiothérapeutique.

14. Vecteur destiné à être utilisé selon l'une quelconque des revendications 1 à 13, dans lequel le vecteur comprend en outre un ou plusieurs fragments de ciblage, éventuellement
dans lequel le ou les fragments de ciblage sont spécifiques à la cellule, éventuellement en outre
dans lequel le ou les fragments de ciblage sont un peptide, un anticorps, un fragment d'anticorps ou un agent thérapeutique.

15. Vecteur destiné à être utilisé selon l'une quelconque des revendications précédentes, le procédé comprenant en outre l'administration d'un deuxième champ électrique alternatif à une deuxième fréquence, dans lequel, éventuellement, le deuxième champ électrique alternatif est un champ de traitement des tumeurs.

16. Vecteur destiné à être utilisé selon l'une quelconque des revendications précédentes, dans lequel l'étape d'introduction du vecteur commence à un moment donné, et dans lequel l'étape d'application du premier champ électrique alternatif se termine au moins 12 heures après le moment donné.

17. Vecteur destiné à être utilisé selon la revendication 16, dans lequel l'étape d'application du premier champ électrique alternatif commence au moins une heure avant le moment donné.

18. Vecteur destiné à être utilisé selon l'une quelconque des revendications précédentes, le procédé comprenant en outre l'administration d'un agent thérapeutique.
